(19) Europäisches Patentamt
European Patent Office
Office européen des brevets

(11) EP 0 389 368 B1

(12) **FASCICULE DE BREVET EUROPEEN**

(45) Date de publication et mention de la délivrance du brevet:
**29.01.1997 Bulletin 1997/05**

(51) Int Cl.[6]: **C07J 43/00**, C07D 213/74, A61K 31/58

(21) Numéro de dépôt: **90400782.0**

(22) Date de dépôt: **22.03.1990**

(54) **Nouveaux dérivés de la 6-(1-pipérazinyl) 2,5-pyridine diamine N,N,N',N'-substituée, leur procédé de préparation et les intermédiaires de ce procédé, leur application comme médicaments et les compositions pharmaceutiques les renfermant**

Derivate von N,N,N',N'-substituiertem 6-(1-Piperazinyl)-2,5-pyridin-diamin, Verfahren zu deren Herstellung und ihre Verwendung als Arzneimittel und pharmazeutische Präparate davon

New derivatives of 6-(1-piperazinyl) 2,5-pyridine diamine N,N,N',N'-substituted, procedure for their production and intermediates, their use as medicines and pharmaceutical preparations containing them

(84) Etats contractants désignés:
**CH DE FR GB IT LI NL**

(30) Priorité: **22.03.1989 FR 8903740**

(43) Date de publication de la demande:
**26.09.1990 Bulletin 1990/39**

(73) Titulaire: **ROUSSEL UCLAF**
**93230 Romainville (FR)**

(72) Inventeurs:
- **Claussner, André**
**F-93250 Villemomble (FR)**
- **Leclaire, Jacques**
**F-91300 Massy (FR)**
- **Nedelec, Lucien**
**F-93340 Le Raincy (FR)**
- **Philibert, Daniel**
**F-94210 La Varenne Saint Hilaire (FR)**

(74) Mandataire: **Vieillefosse, Jean-Claude et al**
**ROUSSEL UCLAF,**
**Département des Brevets,**
**111, Route de Noisy**
**93235 Romainville Cédex (FR)**

(56) Documents cités:
**EP-A- 0 244 115**
**WO-A-87/01706**
**WO-A-87/07895**
**US-A- 4 442 103**

Remarques:
Le dossier contient des informations techniques présentées postérieurement au dépôt de la demande et ne figurant pas dans le présent fascicule.

EP 0 389 368 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux dérivés de la 6-(1-pipérazinyl) 2,5-pyridine diamine N,N,N',N'-substituée, leur procédé de préparation et les intermédiaires de ce procédé, leur application comme médicaments et les compositions pharmaceutiques les contenant.

La demande de brevet internationale WO 87/1706 décrit des stéroides aminés substitués variés, notamment en position 21 et dont l'amine libre correspondante est en particulier une pipérazinylpyridine diamine éventuellement substituée. Ces aminostéroïdes sont différents de ceux de la présente demande ou ne peuvent être obtenus selon le mode opératoire donné lorsque l'amine libre de départ nécessaire est une 6-(1-pipérazinyl) 2,5-pyridine diamine substituée.

Par ailleurs, la demande de brevet internationale WO 87/07895 décrit des aminoesters de type androstane ou de type corticoïde, la demande de brevet européen EP 0244115 concerne des dérivés de la phénylpipérazine et le brevet américain US 4,442,103 décrit des dérivés de la pyridinylpipérazine dont le radical pyridinyl est éventuellement monosubstitué.

Les stéroïdes aminés décrits dans cet art antérieur sont différents des dérivés de la 6-(1-pipérazinyl)-2,5-pyridine diamine N,N,N',N'-substituée de la présente demande.

L'invention a pour objet, les composés de formule (I) :

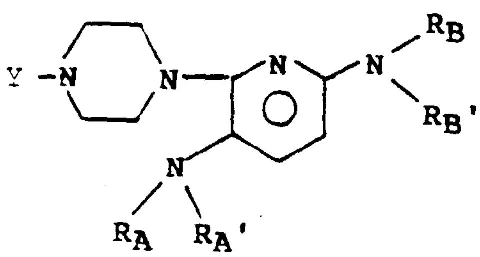

(I)

dans laquelle :

- $R_A$ et $R_A'$, $R_B$ et $R_B'$, identiques ou différents, représentent un atome d'hydrogène ou un radical alkyle ayant de 1 à 4 atomes de carbone,étant entendu que lorsque $R_A$ et $R_{A'}$ sont un atome d'hydrogène ou un radical méthyle, $R_B$ et $R_{B'}$ ne peuvent pas être un radical éthyle; ou forment avec l'atome d'azote auquel ils sont liés un hétérocycle à 5 ou 6 chaînons, éventuellement substitué par un radical alkyle ayant de 1 à 3 atomes de carbone,
  et dans laquelle Y représente un atome d'hydrogène ou le radical

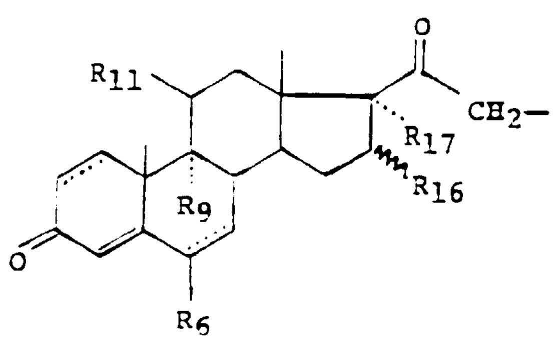

dans lequel :

- $R_6$ représente un atome d'hydrogène, un radical méthyle, un atome de fluor ou un atome de chlore,
- $R_9$ et $R_{11}$ forment ensemble une seconde liaison entre les carbones $C_9$ et $C_{11}$ ou $R_9$ est un atome d'hydrogène ou un atome de fluor et $R_{11}$ est un atome d'hydrogène, un radical hydroxyle ou un radical oxo,
- $R_{16}$ représente un atome d'hydrogène ou un radical méthyle,
- $R_{17}$ représente un atome d'hydrogène, un radical hydroxyle ou un radical acyloxy,
- les traits pointillés, dans les cycles A et B, indiquent la présence éventuelle d'une seconde liaison en position 1 (2) et 6(7),

- le trait ondulé en position 16 indique que le substituant $R_{16}$ peut se trouver en position alpha ou béta, et les sels de ces composés.

Lorsque $R_{17}$ représente un radical acyloxy, il peut s'agir du radical acétyloxy, propionyloxy ou benzoyloxy. Lorsque $R_A$, $R_A'$, $R_B$ ou $R_B'$ représentent un radical alkyle, il peut s'agir des radicaux méthyle, éthyle, n-propyle, isopropyle, n-butyle, isobutyle, sec-butyle, tert-butyle ; il s'agit toutefois de préférence du radical méthyle ou éthyle.

Lorsque $R_A$ et $R_A'$ et/ou $R_B$ et $R_B'$ forment avec l'atome d'azote auquel ils sont liés un hétérocycle, il s'agit d'un hétérocycle saturé, de préférence une pyrrolidine ou une pipéridine, éventuellement substitué par un radical alkyle tel qu'un radical méthyle, éthyle, propyle ou isopropyle, de préférence méthyle ou éthyle, ou d'un hétérocycle insaturé, de préférence un pyrrole, éventuellement substitué par un radical alkyle tel qu'un radical méthyle.

L'invention s'étend naturellement aux sels des composés de formule (I), comme par exemple les sels formés avec les acides chlorhydrique, bromhydrique, nitrique, sulfurique, phosphorique, acétique, formique, propionique, maléique, fumarique, succinique, tartrique, citrique, oxalique, glyoxylique, aspartique, alcanesulfoniques tels que les acides méthane ou éthanesulfoniques, arènesulfoniques, tels que les acides benzène ou paratoluène sulfoniques et arylcarboxyliques, tels que l'acide benzoïque.

Parmi les composés de l'invention, on peut citer en particulier les composés de formule (I) répondant à la formule $(I_A)$ :

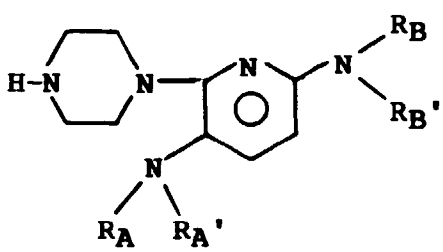

$(I_A)$

dans laquelle $R_A$, $R_{A'}$, $R_B$ et $R_{B'}$ ont la signification indiquée précédemment.

Parmi les composés de l'invention, on peut citer également en particulier les composés de formule (I) répondant à la formule $(I_B)$ :

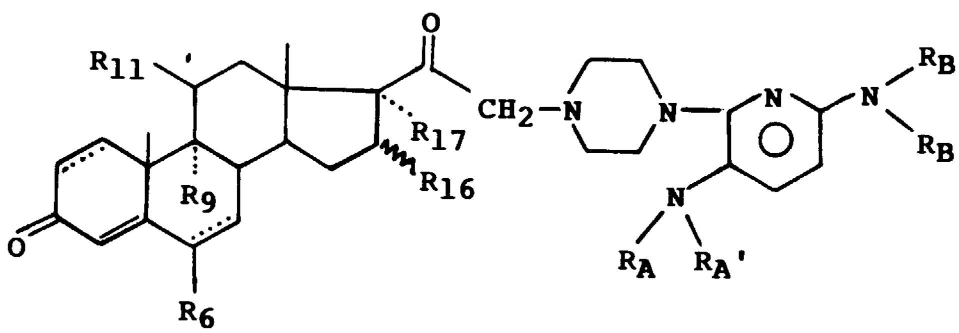

$(I_B)$

dans laquelle $R_6$, $R_9$, $R_{11}$, $R_{16}$, $R_{17}$, $R_A$, $R_{A'}$, $R_B$ et $R_{B'}$, les traits pointillés et le trait ondulé ont la signification indiquée précédemment.

Parmi les composés de l'invention, on peut citer notamment les composés de formule $(I_B)$ pour lesquels $R_6$ est un atome d'hydrogène.

Parmi les composés de l'invention, on peut citer particulièrement les composés de formule $(I_B)$ pour lesquels $R_9$ et $R_{11}$ forment ensemble une seconde liaison entre les carbones qui les portent et ceux pour lesquels $R_{16}$ est un radical méthyle en position alpha.

L'invention a plus particulièrement pour objet les composés de formule $(I_B)$ pour lesquels $R_{17}$ est un atome d'hydrogène ainsi que ceux pour lesquels le trait pointillé en position 1(2) indique la présence d'une seconde liaison.

L'invention a tout particulièrement pour objet les composés dont la préparation est donnée plus loin dans la partie expérimentale à savoir la N,N,N′,N′-tétraéthyl 6-(1-pipérazinyl) 2,5-pyridine diamine et la 21-[4-[3,6-bis (diéthylamino) 2-pyridyl] 1-pipérazinyl] 16alpha-méthyl pregna-1,4,9(11)-triène-3,20-dione et leurs sels.

L'invention a également pour objet un procédé de préparation des composés de formule (I) caractérisé en ce que

l'on fait agir un produit de formule (II) :

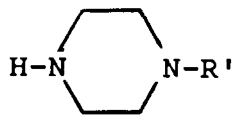

(II)

dans laquelle R′ représente un groupement protecteur de la fonction amino, sur un produit de formule (III) :

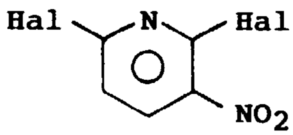

(III)

dans laquelle Hal représente un atome d'halogène, pour obtenir un produit de formule (IV) :

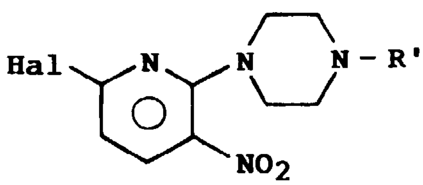

(IV)

sur lequel on fait réagir un produit de formule (V) :

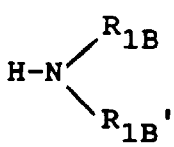

(V)

dans laquelle $R_{1B}$ et $R_{1B}'$ ont soit la signification indiquée ci-dessus pour $R_B$ et $R_B'$ soit sont tels que, ou bien l'un représente un groupement protecteur monovalent de la fonction amino, et l'autre représente un atome d'hydrogène, ou bien $R_{1B}$ et $R_{1B}'$ représentent ensemble un groupement protecteur divalent, pour obtenir un produit de formule (VI) :

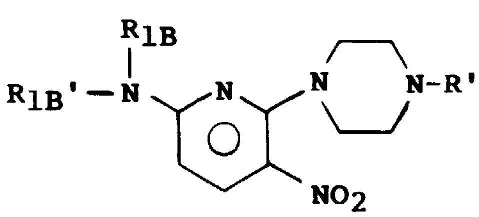

(VI)

que l'on soumet à une réaction d'hydrogénation pour obtenir un produit de formule (VII) :

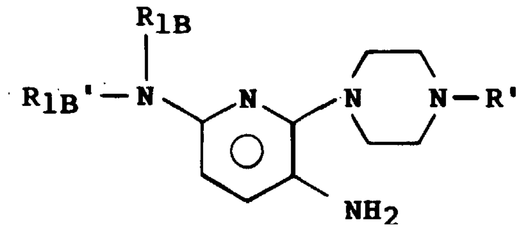

(VII)

produit que, si désiré
**soit** a) l'on soumet à l'action d'un ou deux équivalents d'un dérivé monohalogéné de $R_A$ ou de $R_A'$ pour obtenir un produit de formule (VII′) :

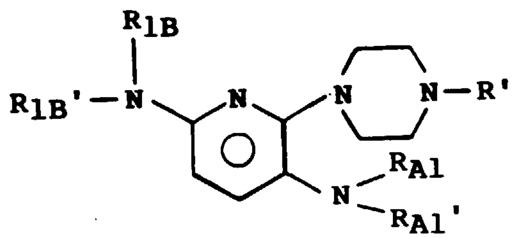

(VII')

dans laquelle $R_{A1}$ et $R_{A1}'$ représentent soit l'un un atome d'hydrogène et l'autre un radical alkyle, soit tous les deux le même radical alkyle,
**soit** b) l'on soumet à l'action d'un dérivé monohalogéné de $R_A$ ou de $R_A'$, puis à l'action d'un dérivé monohalogéné de $R_A'$ ou de $R_A$ pour obtenir un produit de formule (VII″) :

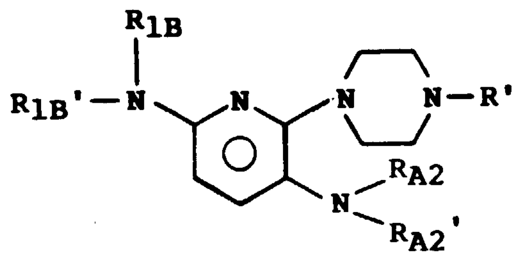

(VII'')

dans laquelle $R_{A2}$ et $R_{A2}'$ représentent des radicaux alkyles différents,
**soit** c) l'on soumet à l'action d'un dérivé dihalogéné du butane ou du pentane, éventuellement substitué par un radical alkyle ayant de 1 à 3 atomes de carbone pour obtenir un produit de formule (VII‴) :

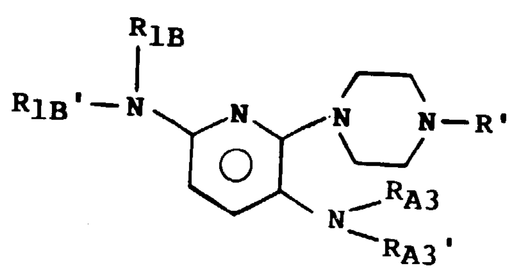

(VII''')

dans laquelle $R_{A3}$ et $R_{A3}'$ forment avec l'atome auquel ils sont liés un hétérocycle à 5 ou 6 chaînons,
produits de formule (VII), (VII′), (VII″) et (VII‴) que l'on soumet à une réaction de déblocage du groupement R′ et si désiré des groupements $R_{1B}$ et/ou $R_{1B}'$ pour obtenir un composé de formule ($I_A$) :

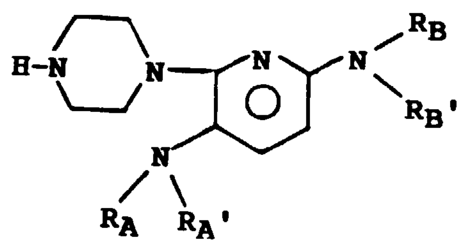

$(I_A)$

dans laquelle $R_A$, $R_A'$, $R_B$ et $R_B'$ ont la signification indiquée précédemment, produit de formule $(I_A)$ que, si désiré, l'on salifie ou si désiré, l'on soumet, dans un solvant neutre et en présence d'une base, à l'action d'un composé de formule (VIII) :

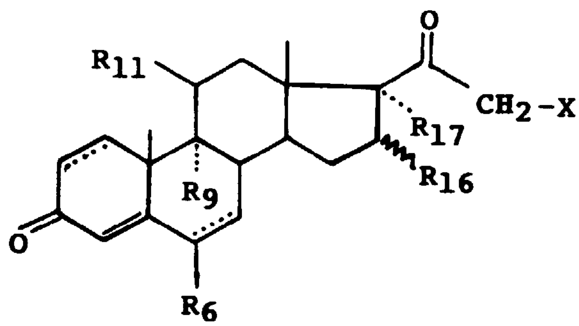

(VIII)

dans laquelle X est un atome d'halogène et $R_6$, $R_9$, $R_{11}$, $R_{16}$, $R_{17}$, les traits pointillés et le trait ondulé ont la même signification que précédemment, pour obtenir un composé de formule $(I_B)$ :

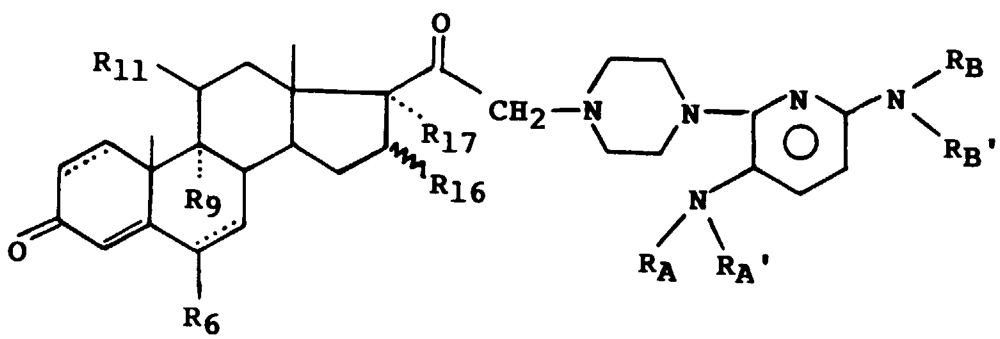

$(I_B)$

et si désiré, l'on soumet le composé obtenu à l'action d'un acide pour obtenir le sel correspondant.

Dans un mode de réalisation préféré du procédé de l'invention, les composés de formule $(I_A)$ sont obtenus en faisant réagir un produit de formule (II) dont le groupement protecteur de la fonction amino est par exemple un radical acétyle sur un produit de formule (III) dont le substituant Hal représente par exemple un atome de chlore ; dans le produit de formule (V), le groupement protecteur monovalent de la fonction amino que peut représenter $R_{1B}$ ou $R_{1B'}$ est par exemple un groupement benzyle ou trityle : lorsque $R_{1B}$ et $R_{1B'}$ représentent ensemble un groupement divalent, ils peuvent par exemple former ensemble avec l'atome d'azote auquel ils sont liés un 2,5-diméthylpyrrole.

Les composés de formule $(I_B)$ sont obtenus en faisant réagir un composé de formule (VIII) dont le substituant halogéné X peut être un atome de chlore, de brome ou d'iode, dans un solvant neutre tel que par exemple le diméthylformamide, le tétrahydrofuranne, le chlorure de méthylène, l'acétonitrile, l'éther éthylique ou l'acétone, en présence d'une base telle que par exemple un carbonate ou un bicarbonate de métal alcalin, de préférence de sodium ou de potassium, la triéthylamine ou la diisopropyléthylamine avec la pipérazine N-substituée de formule $(I_A)$.

Dans un mode de réalisation préféré du procédé de l'invention, le composé de formule (VIII) est le 21-iodo 16alpha-méthyl pregna-1,4,9(11)-triène-3,20-dione décrit dans la demande de brevet WO 87/01706, le solvant neutre est l'acétone, la base est le carbonate de potassium.

Les composés de formule (I) concernés par l'invention peuvent être obtenus à l'état de sels par des méthodes

connues qui consistent à faire réagir le composé (I) avec un acide minéral ou organique choisi dans la liste des acides indiquée précédemment.

Les acides préférés, selon l'invention, sont l'acide méthane-sulfonique ou l'acide fumarique.

Les composés de formule (I) ainsi que leurs sels présentent d'intéressantes propriétés pharmacologiques, notamment, une activité antioxydante par inhibition de la péroxydation lipidique tissulaire, par exemple au niveau du rein, du coeur, plus particulièrement au niveau du cerveau et de la moelle épinière. Les composés de formule (I) ainsi que leurs sels présentent aussi une activité détoxifiante dans les intoxications aigues associées à la péroxydation des lipides des tissus cérébraux tels que le cerveau ou la moelle épinière.

Les composés de formule (I) ainsi que leurs sels présentent en outre une activité anti-inflammatoire intéressante, par exemple dans les phénomènes de l'inflammation aigüe médiée par les dérivés de l'acide arachidonique.

Ces propriétés justifient leur application en thérapeutique.

L'invention a donc pour objet les composés de formule (I) ainsi que leurs sels avec les acides pharmaceutiquement acceptables, à titre de médicaments.

Parmi les médicaments de l'invention, on peut citer tout particulièrement les composés des exemples, à savoir : la N,N,N′,N′-tétraéthyl 6-(1-pipérazinyl) 2,5-pyridine diamine, et la formule 21-[4-[3,6-bis(diéthylamino) 2-pyridyl] 1-pipérazinyl] 16alpha-méthyl pregna-1,4,9(11)-triène-3,20-dione et leurs sels avec les acides pharmaceutiquement acceptables.

Les médicaments selon l'invention peuvent être utilisés dans le traitement de désordres biologiques consécutifs à des traumatismes. On entend, par traumatismes, des dommages tissulaires dans lesquels interviennent la génération de péroxydes lipidiques et qui peuvent être provoqués par des agents variés, par exemple des agents physiques tels que des contusions, en particulier des contusions cérébrales associées ou non à des hémorragies locales, ou des agents chimiques tels que ceux utilisés en chimiothérapie antitumorale comme l'adriamycine ou tels que ceux utilisés en immunothérapie cancéreuse comme l'IL2 ou le TNF.

Ils sont surtout intéressants dans le traitement de l'ischémie cérébrale, en particulier dans le traitement de l'infarctus cérébral et dans la prévention de sa récidive, ou dans le traitement d'intoxications médicamenteuses provoquées par une chimiothérapie ou une immunothérapie ou l'association des deux.

Ils peuvent en outre être utilisés dans le traitement des réactions inflammatoires ; ils sont intéressants pour le traitement des réactions inflammatoires locales, comme par exemple les oedèmes, les dermatoses, les prurits, les diverses formes d'eczéma et les érythèmes solaires ou pour le traitement de maladies inflammatoires aigues ou les maladies inflammatoires chroniques, par exemple la polyarthrite rhumatoïde, le psoriasis ou la sclérose en plaque.

Les médicaments de l'invention peuvent être administrés par voie orale, par voie parentérale, telle qu'en injection intra-musculaire, intra-articulaire, intrathécale et de préférence en injection intraveineuse en bolus ou en perfusion continue ou par voie locale en application topique sur la peau ou les muqueuses.

L'invention s'étend aux compositions pharmaceutiques renfermant comme principe actif au moins un médicament tel que défini ci-dessus.

Les compositions pharmaceutiques sont préparées selon les méthodes usuelles. Le principe actif peut être incorporé à des excipients habituellement employés dans ces compositions pharmaceutiques, tels que le talc, l'amidon, les véhicules aqueux ou non, les corps gras d'origine animale ou végétale, les dérivés paraffiniques, les glycols, les divers agents mouillants, dispersants ou émulsifiants et les conservateurs.

Les sels pharmaceutiquement acceptables des produits de formule (I), plus solubles dans l'eau, sont utilisés de préférence pour les formulations aqueuses destinées à l'injection intraveineuse.

La posologie utile, la fréquence et la durée du traitement qui dépendent du composé et de la voie d'administration choisis varient, notamment en fonction du sujet et de la sévérité de l'affection à traiter. La dose peut être comprise entre 0,02 et 100 mg/kg/j, de préférence entre 0,01 et 1 mg/kg/j par voie intraveineuse ou par voie intramusculaire et éventuellement répétée plusieurs fois par jour.

Les composés de formule (VIII) utilisés dans le procédé de l'invention sont des 21-halo stéroïdes qui peuvent être préparés, d'une manière générale, à partir de stéroïdes correspondants 21-hydroxylés selon des méthodes connues de l'homme de métier ou par exemple selon le procédé décrit dans la demande de brevet WO 87/1706.

Les stéroïdes 21-hydroxylés ayant un atome d'hydrogène ou un radical hydroxyle en 17alpha sont des stéroïdes connus ou dont la préparation est connue de l'homme de métier. Ils peuvent, par exemple, être préparés à partir des stéroïdes 17-céto correspondants en utilisant les procédés décrits dans les demandes de brevets FR 2 462 445 et FR 2 498 607. Les stéroïdes 17-céto sont eux-mêmes des produits connus décrits par exemple dans les brevets USP 2.775.602, 2.793.218, 4.189.431, 3.505.365 ou 2.656.370.

Les stéroïdes ayant un radical acyloxy en 17 sont préparés par acylation des steroïdes 17alpha-hydroxylés correspondants.

Les steroïdes ayant une double liaison en 9(11) sont des produits connus ou préparés selon les méthodes connues de l'homme de métier, par exemple par deshydratation d'un stéroïde 11-hydroxylé correspondant par un mélange de chlorure de méthanesulfonyle et de chlorure de thionyle.

Les stéroïdes ayant une double liaison en 1(2) ou en 6(7) sont obtenus à partir d'un dérivé delta-4 correspondant, selon les méthodes connues de l'homme de métier, par exemple par action d'un dérivé de la p. benzoquinone tel que le chloranile ou la 2,3-dichloro 5,6-dicyanobenzoquinone (DDQ). Les dérivés ayant une double liaison en 1(2) peuvent être aussi obtenus par voie biochimique à l'aide de microorganismes tel que Arthrobacter Simplex.

Les stéroïdes ayant un radical méthyle en $C_6$ sont préparés selon des méthodes connues de l'homme de métier, par exemple par action d'un halogénure de méthylmagnésium sur un 5(6)- époxy stéroïde correspondant ayant une cétone en position 3 bloquée par exemple par un groupement cétal.

Les stéroïdes ayant un atome de fluor en $C_6$ sont préparés selon les méthodes connues, par exemple par addition du complexe acide fluorhydrique-diméthylformamide sur un 5(6)- époxy stéroïde correspondant ayant une cétone en position 3 bloquée par exemple par un groupement cétal.

Les stéroïdes ayant un atome de chlore en $C_6$ sont préparés selon des méthodes connues, par exemple par addition du complexe acide chlorhydrique-diméthylformamide sur un 5(6)-époxy stéroïde correspondant ayant une cétone en position 3 bloquée par exemple par un groupement cétal.

Les stéroïdes ayant un radical méthyle en $C_{16}$ alpha sont préparés selon des méthodes connues, par exemple par addition d'un halogènure de méthylmagnésium en présence d'un sel de cuivre sur un delta-16 20-céto stéroïde correspondant, lui-même obtenu par déshydratation du stéroïde 17-hydroxylé correspondant.

Les stéroïdes ayant un radical méthyle en $C_{16}$ béta sont préparés selon des méthodes connues, par exemple à partir d'un delta-16 20-céto stéroïde correspondant que l'on traite au diazométhane, chauffe puis soumet à une réaction d'hydrogénation.

Les composés de formule (VII), (VII′), (VII″) et (VII‴) sont des produits nouveaux et l'invention a donc également pour objet les composés de formule (VII), (VII′), (VII″) et (VII‴) à titre de produits industriels nouveaux.

Les produits (VII), (VII′), (VII″) et (VII‴), ainsi que tous les produits de formule ($I_A$) contrairement à ce qui est indiqué, ne peuvent pas être obtenus selon la préparation décrite dans la demande de brevet WO 87/01706.

Les exemples suivants illustrent l'invention sans toutefois la limiter.

**Exemple 1 : N,N,N′,N′-tétraéthyl 6-(1-pipérazinyl) 2,5-pyridine diamine.**

Stade A : 1-acétyl 4-[6-(diéthylamino) 3-nitro 2-pyridyl] pipérazine

A un mélange de 78 g de 2,6-dichloro 3-nitro pyridine, 600 $cm^3$ d'acétonitrile et 66,3 g de carbonate de potassium, on ajoute en 50 minutes à 0°C, une solution de 51,22 g de N-acétyl pipérazine dans 200 $cm^3$ d'acétonitrile. On laisse revenir à température ambiante et agite 1 heure 15. On filtre les sels minéraux, ajoute au filtrat, 180 $cm^3$ de N-diéthylamine et 76 g de carbonate de potassium et chauffe le mélange 1 heure 15 au reflux. Après refroidissement, on filtre les sels minéraux, et évapore à sec sous pression réduite, le résidu (166,9 g) est recristallisé dans 200 $cm^3$ d'acétate d'éthyle, on obtient 87,4 g de produit attendu. F = 120°C.

| Spectre IR ($CHCl_3$) | |
|---|---|
| C = O | 1637 $cm^{-1}$ |
| Système conjugué | 1593 $cm^{-1}$ |
| 1ère bande $NO_2$ | 1569 $cm^{-1}$ - 1510 $cm^{-1}$ |
| 2ème bande $NO_2$ | 1346 ou 1299 |

Stade B : N,N,N′,N′-tétraéthyl 6-(1-pipérazinyl) 2,5-pyridine-diamine.

On hydrogène sous une pression maximum de 1250 mbar à 25°C, un mélange de 70 g du produit obtenu au stade A, 1500 $cm^3$ de méthanol, 61,5 $cm^3$ d'acétaldéhyde et 10 g de charbon actif à 10 % de palladium. On absorbe environ 20 litres d'hydrogène, on filtre le catalyseur et évapore le filtrat à sec sous pression réduite. On reprend le résidu (89,8 g) avec 500 $cm^3$ de n-propanol et 91,3 g de potasse en pastilles, et chauffe 3 heures au reflux. On coule la solution refroidie dans 1 litre d'eau glacée et extrait avec du chlorure de méthylène, lave la solution organique avec une solution saturée de chlorure de sodium, sèche, filtre et concentre à sec, sous pression réduite. On chromatographie le résidu (58,9 g) sur silice (éluant : chlorure de méthylène-méthanol-ammoniaque (95-5-0,5)). On obtient 48,35 g de produit attendu.

| Analyse pour $C_{17}H_{31}N_5$ | | | |
|---|---|---|---|
| Calculé : | C% 66,84 | H% 10,23 | N% 22,93 |
| Trouvé : | 66,9 | 10,5 | 22,6 |

| Spectre IR ($CHCl_3$) | |
|---|---|
| C = C<br>C = N<br>Hétéroaromatique | 1596 $cm^{-1}$<br>1560 $cm^{-1}$<br>1531 $cm^{-1}$<br>1487 $cm^{-1}$ |

**<u>EXEMPLE 2</u> : 21-[4-[3,6-bis(diéthylamino) 2-pyridyl] 1-pipérazinyl] 16alpha-méthyl pregna-1,4,9(11)-triène-3,20-dione.**

On mélange à température ambiante 1,395 g de 21-iodo 16alpha-méthyl pregna-1,4,9(11)-triène-3,20-dione (préparé selon brevet WO 87/01706) 40 $cm^3$ d'acétone, 1,88 g de produit obtenu à l'exemple 1 et 0,9 g de carbonate de potassium. On agite pendant 2 heures 30, filtre et amène le filtrat à sec sous pression réduite. On chromatographie le résidu (3,47 g) sur silice (éluant : cyclohexane-acétate d'éthyle (7-3)). On obtient 1,4 g de produit attendu.
$[alpha]_D$ = +41° ± 2°5 (c = 0,4 % éthanol).

| Analyse pour $C_{39}H_{57}N_5O_2$ | | | |
|---|---|---|---|
| Calculé : | C% 74,6 | H% 9,15 | N% 11,15 |
| Trouvé : | 74,5 | 9,4 | 11,5 |

| Spectre IR ($CHCl_3$) | |
|---|---|
| delta-1,4 3-one | 1663 $cm^{-1}$<br>1624 $cm^{-1}$<br>1604 $cm^{-1}$<br>889 $cm^{-1}$ |
| cétone non conjuguée | 1714 $cm^{-1}$<br>1701 $cm^{-1}$ |
| hétéroaromatique | 1594 $cm^{-1}$<br>1561 $cm^{-1}$<br>1486 $cm^{-1}$ |

**<u>ETUDE PHARMACOLOGIQUE</u>**

I) <u>Activité antioxydante.</u>

L'activité antioxydante est recherchée, in vitro, par le test de formation de la malonyldialdéhyde (MDA) qui mesure la péroxydation lipidique déclenchée :
**soit a)** de façon non enzymatique par le sulfate ferreux, dans

1) des homogénats de cerveau ou
2) des microsomes hépatiques de rat

**soit b)** de façon enzymatique par NADPH et le tétrachlorure de carbone, dans des microsomes hépatiques de rat.

**1.1 :** La formation de MDA est mesurée sur des homogénats au 1/10$^e$ (V/V) de cerveaux de rat S.D (200 g) préparés dans un tampon de Krebs pH 7,4 selon les conditions décrites dans J. Biol. Chem. 262 (1987) 10438-10440. On incube 1 ml d'homogénat pendant 60 mn à 37°C en présence de 25 microlitres d'éthanol contenant ou non le produit à tester, après addition de 25 microlitres de solution de sulfate ferreux préparée extemporanément dans de l'eau dégazée à l'argon (200 micromoles final). On prélève 0,25 ml de mélange incubé et ajoute 1,5 ml d'acide phosphorique à 1 %, 0,25 ml de solution 200 micromoles de déféroxamine (Desféral$^R$ Ciba Geigy) dans l'eau, 10 microlitres de tert-butylhydroxytoluène (BHT) à 8,7 mg/ml dans l'éthanol et 0,5 ml d'acide thiobarbiturique (TBA) à 0,6 % dans l'eau. On chauffe le mélange à 100°C pendant 45 mn, refroidit, ajoute 4 ml de n-butanol, centrifuge pendant 15 mn à 4000 t/mn puis lit la DO à 535 nm de la fraction surnageante. On incube dans les mêmes conditions

les blancs de réaction en absence de Fe++. On calcule le pourcentage d'inhibition :

$$\text{pourcentage d'inhibition} = \frac{\text{DO en présence de produit}}{\text{DO en absence de produit}}$$

Résultats :

| concentration | $5.10^{-4}$M | $1.10^{-4}$M | $1.10^{-5}$M |
|---|---|---|---|
| pourcentage d'inhibition | | | |
| Produit de l'ex. 1 | 74,2 ± 3,8 | 67,4 ± 8,6 | 60,3 ± 9,1 |
| Produit de l'ex. 2 | 60,4 ± 4,4 | 55,6 ± 1,3 | 63,8 ± 4,3 |

**1.2 :** La formation de MDA est mesurée sur des microsomes hépatiques de rat S.D (200 g) préparés à partir de la fraction sédimentée à 100.000 g d'un homogénat de foie dans un tampon sucrose dont on utilise la fraction restant insoluble à 100.000 g dans un tampon de pyrophosphate de sodium 100 mM pH 7,4 et que l'on homogénéise dans un tampon phosphate de sodium 100 mM pH 7,4 à 20 % de glycérol, puis que l'on conserve à -80°C.

On incube les microsomes pendant 15 mn à 37°C dans 1 ml contenant du tampon Tris-HCl 35 mM, KCl 0,1 M pH 7,4, 1 mg de protéine microsomale, 5 microlitres d'éthanol renfermant ou non le produit à tester, 250 microlitres de solution d'ascorbate dans le tampon Tris (0,5 mM final), après l'addition de 250 microlitres de sulfate ferreux préparée extemporanément dans le tampon Tris (6 micromoles final). On arrête la réaction par addition de 2 ml d'une solution d'acide trichloracétique 1 M dans l'acide chlorhydrique 0,25 M contenant 0,4 % d'acide thiobarbiturique. On chauffe le mélange à 85°C pendant 25 mn, refroidit, centrifuge pendant 15 mn à 3500 t/mn puis lit la DO à 535 nm de la fraction surnageante. On effectue en même temps les blancs de réaction en absence de Fe++. On calcule le pourcentage d'inhibition comme précédemment.
Résultats :

| concentration | $1.10^{-5}$M | $5.10^{-6}$M | $1.10^{-6}$M |
|---|---|---|---|
| pourcentage d'inhibition | | | |
| Produit de l'ex. 1 | 99,4 | 99,4 | 44,0 |
| Produit de l'ex. 2 | 99,8 | 99,5 | 42,0 ± 5 |

**2 :** La formation de MDA est mesurée sur des microsomes hépatiques de rats prétraités au phénobarbital (80 mg/kg par 3 injections i.p), préparés comme décrit précédemment. On incube les microsomes pendant 15 mn à 37°C dans 1 ml contenant du tampon phosphate 0,1 M pH 7,4, 1 mg de protéine microsomale, 5 microlitres d'éthanol renfermant ou non le produit à tester, 5 microlitres de tétrachlorure de carbone (5,5 mM final) après l'addition de 50 microlitres de solution de NADPH dans le tampon phosphate (1 mM final). On arrête la réaction puis effectue le dosage selon les conditions décrites précédemment.

Résultats :

| concentration | $1.10^{-5}$M | $5.10^{-6}$M | $1.10^{-6}$M |
|---|---|---|---|
| pourcentage d'inhibition | | | |
| Produit de l'ex. 1 | 96 ± 1 | 93 ± 0,2 | 43 ± 3 |
| Produit de l'ex. 2 | 91 ± 1 | 88 ± 1 | 49 ± 1,5 |

II) <u>Activité anti-inflammatoire.</u>

L'activité anti-inflammatoire est évaluée in vivo par mesure de l'activité anti-oedémateuse à l'aide du test de l'oedème plantaire provoqué par l'acide arachidonique décrit par M.J. Di Martino et al. (Agents and Actions 1987 21 3/4 303).

Les animaux d'expérimentation sont des rats mâles EOPS de souche Sprague Dawley pesant 150-170 g (Iffa Credo).

Ce test est pratiqué sur des groupes de 8 rats mâles pesant 130 à 150 g à jeun depuis 16 heures. L'acide arachidonique est injecté sous l'aponévrose plantaire d'une patte postérieure à la dose de 0,2 mg sous un volume de 0,1

ml. Le volume de la patte est mesuré à l'aide d'un pléthysmomètre à eau, avant et 1 heure après l'injection de l'acide arachidonique. La différence entre ces deux volumes représente le degré d'inflammation. Les animaux sont traités par les produits à étudier ou le véhicule seul en même temps que l'injection de l'acide arachidonique.

Les produits étudiés ont été administrés par voie orale sous un volume de 4 ml/kg après avoir été mis en suspension dans la méthylcellulose à 0,5%. Toutes les expériences ont été réalisées avec comme produit de référence la dexaméthasone à la dose de 0,5 mg/kg par voie orale.

Les résultats sont exprimés en variation de volume de la patte 1 heure après l'injection d'acide arachidonique (AA), en absence ou en présence du produit à tester administré à différentes doses.

L'interprétation statistique des résultats a été effectuée selon le test de Dunnett (* $p < 0,05$ - ** $p < 0,01$) ou selon le test de Mann Whitney (° $p < 0,05$ - °° $p < 0,01$).

Pour chaque dose administrée par voie orale, on calcule le pourcentage d'inhibition de l'oedème par le produit testé, par rapport au témoin.

| | Dose mg/kg | Variation de volume de la patte 1 heure après AA(cm3) | % inhibition |
|---|---|---|---|
| Témoins | 0 | 0,58 ± 0,04 | |
| Produit de l'exemple 2 | 0,5 | 0,32 ± 0,04** | - 45 |
| | 1 | 0,32 ± 0,01** | - 45 |
| Témoins | 0 | 0,63 ± 0,01 | |
| Produit de l'exemple 1 | 20 | 0,33 ± 0,06** | - 48 |

Les produits étudiés ont une activité anti-inflammatoire a faible dose, en particulier le produit de l'exemple 2 qui présente une forte activité dès la dose de 0,5 mg/kg.

Dans les mêmes conditions expérimentales, la dexaméthasone induit une inhibition de l'oedème de 30 à 40% à la dose de 0,5 mg/kg.

**Revendications**

**1.** Les composés de formule (I) :

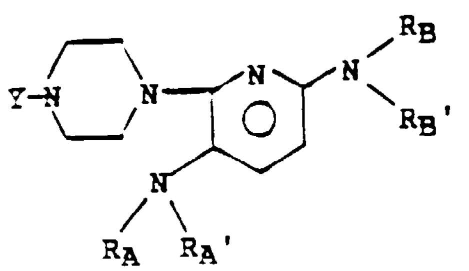

(I)

dans laquelle :

- $R_A$ et $R_A'$, $R_B$ et $R_B'$, identiques ou différents, représentent un atome d'hydrogène ou un radical alkyle ayant de 1 à 4 atomes de carbone, étant entendu que lorsque $R_A$ et $R_{A'}$ sont un atome d'hydrogène ou un radical méthyle, $R_B$ et $R_{B'}$ ne peuvent pas être un radical éthyle ; ou forment avec l'atome d'azote auquel ils sont liés un hétérocycle à 5 ou 6 chaînons, éventuellement substitué par un radical alkyle ayant de 1 à 3 atomes de carbone,

  et dans laquelle Y représente le radical

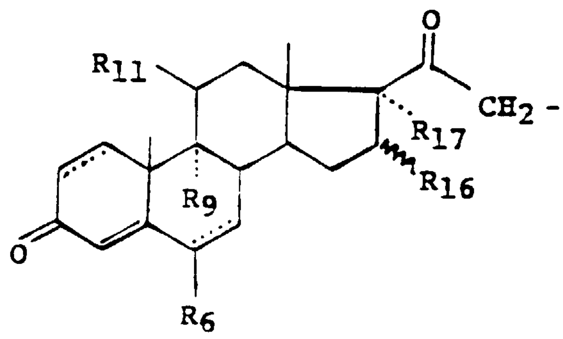

dans lequel :

- $R_6$ représente un atome d'hydrogène, un radical méthyle, un atome de fluor ou un atome de chlore,
- $R_9$ et $R_{11}$ forment ensemble une seconde liaison entre les carbones $C_9$ et $C_{11}$ ou $R_9$ est un atome d'hydrogène ou un atome de fluor et $R_{11}$ est un atome d'hydrogène, un radical hydroxyle ou un radical oxo,
- $R_{16}$ représente un atome d'hydrogène ou un radical méthyle,
- $R_{17}$ représente un atome d'hydrogène, un radical hydroxyle ou un radical acyloxy,
- les traits pointillés, dans les cycles A et B, indiquent la présence éventuelle d'une seconde liaison en position 1(2) et 6(7),
- le trait ondulé en position 16 indique que le substituant $R_{16}$ peut se trouver en position alpha ou béta, et les sels de ces composés.

**2.** Les composés de formule (I) telle que définie à la revendication 1, pour lesquels $R_6$ est un atome d'hydrogène.

**3.** Les composés de formule (I) telle que définie à la revendication 1 ou 2, pour lesquels $R_9$ et $R_{11}$ forment ensemble une seconde liaison entre les carbones qui les portent.

**4.** Les composés de formule (I) telle que définie à l'une quelconque des revendications 1 à 3, pour lesquels $R_{16}$ est un radical méthyle en position alpha.

**5.** Les composés de formule (I) telle que définie à l'une quelconque des revendications 1 à 4, pour lesquels $R_{17}$ est un atome d'hydrogène.

**6.** Les composés de formule (I) telle que définie à l'une quelconque des revendications 1 à 5, pour lesquels le trait pointillé en position 1(2) indique la présence d'une seconde liaison.

**7.** Le composé de formule (I) telle que définie à l'une quelconque des revendications 1 à 6, répondant à la formule suivante :

- la 21-[4-[3,6-bis (diéthylamino) 2-pyridyl] 1-pipérazinyl] 16alpha-méthyl pregna-1,4,9(11)-triène-3,20-dione et ses sels.

**8.** Procédé de préparation des composés de formule (I), telle que définie à la revendication 1, caractérisé en ce que l'on fait agir un produit de formule (II) :

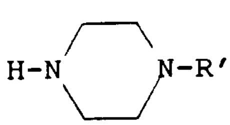

(II)

dans laquelle R' représente un groupement protecteur de la fonction amino, sur un produit de formule (III) :

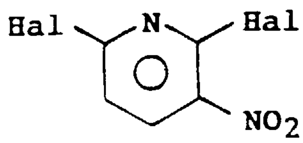

(III)

dans laquelle Hal représente un atome d'halogène, pour obtenir un produit de formule (IV) :

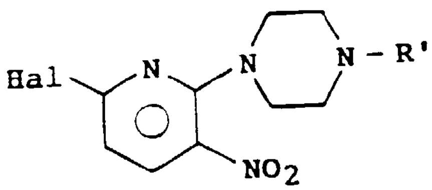

(IV)

sur lequel on fait réagir un produit de formule (V) :

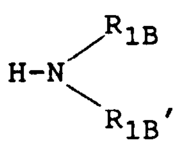

(V)

dans laquelle $R_{1B}$ et $R_{1B}$' ont soit la signification indiquée ci-dessus pour $R_B$ et $R_B$' soit sont tels que, ou bien l'un représente un groupement protecteur monovalent de la fonction amino, et l'autre représente un atome d'hydrogène, ou bien $R_{1B}$ et $R_{1B}$' représentent ensemble un groupement protecteur divalent, pour obtenir un produit de formule (VI) :

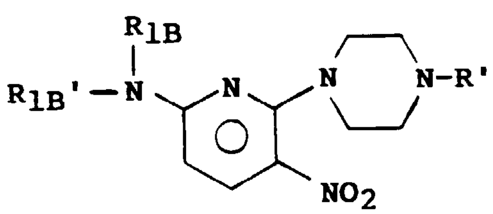

(VI)

que l'on soumet à une réaction d'hydrogénation pour obtenir un produit de formule (VII) :

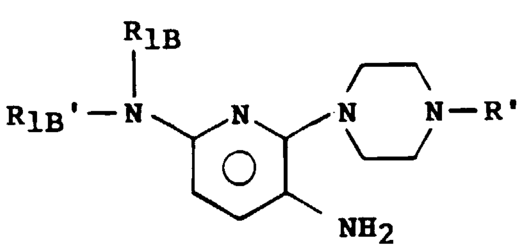

(VII)

produit que, si désiré

**soit** a) l'on soumet à l'action d'un ou deux équivalents d'un dérivé monohalogéné de $R_A$ ou de $R_A$' pour obtenir un produit de formule (VII') :

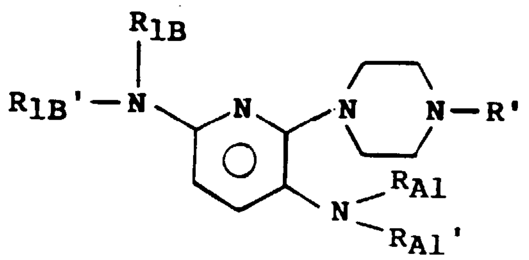

(VII')

dans laquelle $R_{A1}$ et $R_{A1}'$ représentent soit l'un un atome d'hydrogène et l'autre un radical alkyle, soit tous les deux le même radical alkyle,

**soit** b) l'on soumet à l'action d'un dérivé monohalogéné de $R_A$ ou de $R_A'$, puis à l'action d'un dérivé monohalogéné de $R_A'$ ou de $R_A$ pour obtenir un produit de formule (VII") :

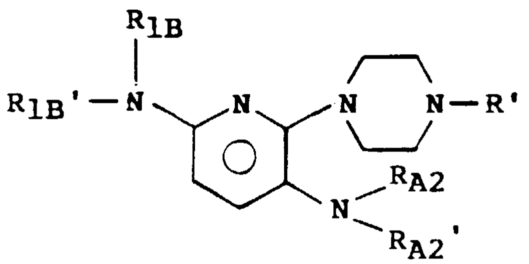

(VII'')

dans laquelle $R_{A2}$ et $R_{A2}'$ représentent des radicaux alkyles différents,

**soit** c) l'on soumet à l'action d'un dérivé dihalogéné du butane ou du pentane, éventuellement substitué par un radical alkyle ayant de 1 à 3 atomes de carbone pour obtenir un produit de formule (VII'''):

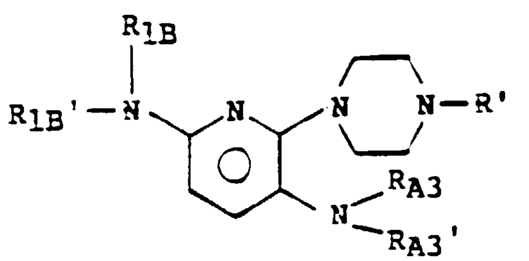

(VII''')

dans laquelle $R_{A3}$ et $R_{A3}'$ forment avec l'atome auquel ils sont liés un hétérocycle à 5 ou 6 chaînons, produits de formule (VII), (VII'), (VII") et (VII''') que l'on soumet à une réaction de déblocage du groupement R' et si désiré des groupements $R_{1B}$ et/ou $R_{1B}'$ pour obtenir un composé de formule ($I_A$) :

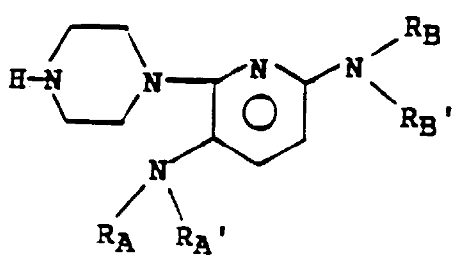

($I_A$)

dans laquelle $R_A$, $R_A'$, $R_B$ et $R_B'$ ont la signification indiquée à la revendication 1, produit de formule ($I_A$) que, si désiré l'on salifie ou si désiré, l'on soumet, dans un solvant neutre et en présence d'une base, à l'action d'un composé de formule (VIII) :

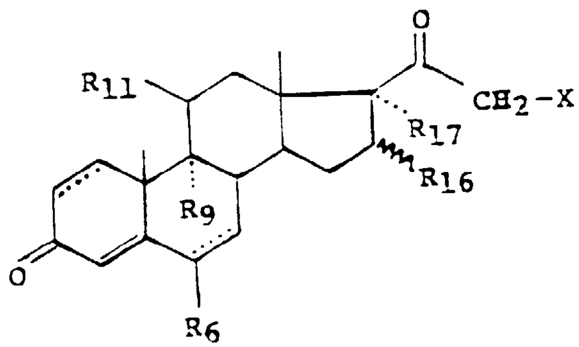

(VIII)

dans laquelle X est un atome d'halogène et $R_6$, $R_9$, $R_{11}$, $R_{16}$, $R_{17}$, les traits pointillés et le trait ondulé ont la même signification que précédemment, pour obtenir un composé de formule (I) :

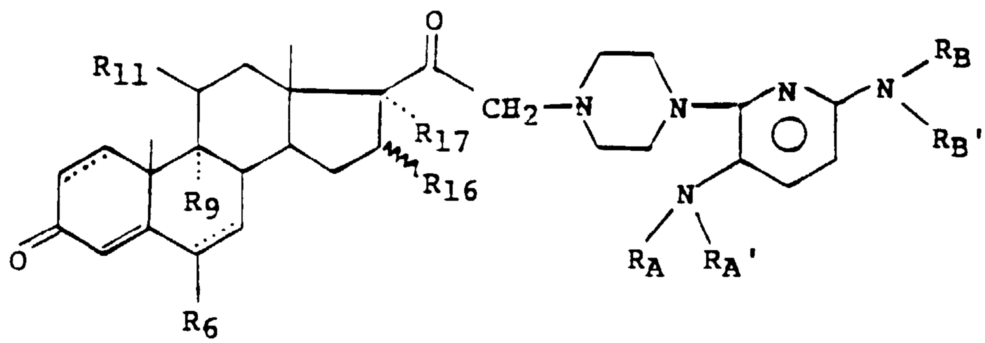

(I)

et si désiré, l'on soumet le composé obtenu à l'action d'un acide pour obtenir le sel correspondant.

**9.** A titre de médicament, les composés de formule (I) tels que définis à l'une quelconque des revendications 1 à 6 et leurs sels avec les acides pharmaceutiquement acceptables.

**10.** A titre de médicament, selon la revendication 9, le composé défini à la revendication 7 et ses sels avec les acides pharmaceutiquement acceptables.

**11.** Les compositions pharmaceutiques renfermant comme principe acif au moins un médicament tel que défini à l'une quelconque des revendications 9 à 10.

**12.** A titre de produits industriels nouveaux, les composés de formule (VII), (VII'), (VII"), (VII"') et ($I_A$).

**Patentansprüche**

**1.** Verbindungen der Formel (I)

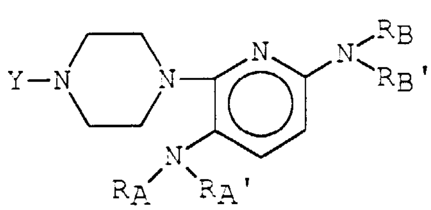

(I)

in der

- $R_A$ und $R_A'$, $R_B$ und $R_B'$, gleich oder verschieden, ein Wasserstoffatom oder einen Alkylrest mit 1 bis 4 Kohlenstoffatomen darstellen, mit der Maßgabe, daß in dem Fall, wo $R_A$ und $R_A'$ ein Wasserstoffatom oder einen Methylrest bedeuten, $R_B$ und $R_B'$ kein Ethylrest sein können; oder zusammen mit dem Stickstoffatom, an das sie gebunden sind, einen Heterocyclus mit 5 oder 6 Ringgliedern bilden, gegebenenfalls substituiert durch einen Alkylrest mit 1 bis 3 Kohlenstoffatomen,

  und in der Y den Rest

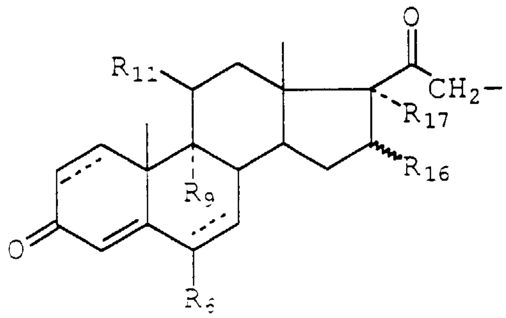

darstellt, worin

- $R_6$ ein Wasserstoffatom, einen Methylrest, ein Fluoratom oder ein Chloratom darstellt,
- $R_9$ und $R_{11}$ zusammen eine zweite Bindung zwischen den Kohlenstoffen $C_9$ und $C_{11}$ bedeuten oder $R_9$ ein Wasserstoffatom oder ein Fluoratom ist und $R_{11}$ ein Wasserstoffatom, ein Hydroxylrest oder ein Oxorest ist,
- $R_{16}$ ein Wasserstoffatom oder einen Methylrest darstellt,
- $R_{17}$ ein Wasserstoffatom, einen Hydroxylrest oder einen Acyloxyrest bedeutet,
- die punktierten Linien in den Ringen A und B die mögliche Anwesenheit einer zweiten Bindung in Position 1 (2) und 6(7) anzeigen,
- die Wellenlinie in Position 16 anzeigt, daß sich der Substituent $R_{16}$ in Position alpha oder beta befinden kann, und die Salze dieser Verbindungen.

2. Verbindungen der Formel (I) wie in Anspruch 1 definiert, worin $R_6$ ein Wasserstoffatom ist.

3. Verbindungen der Formel (I) wie in Anspruch 1 oder 2 definiert, worin $R_9$ und $R_{11}$ zusammen eine zweite Bindung zwischen den Kohlenstoffen bilden, die sie tragen.

4. Verbindungen der Formel (I) wie in irgendeinem der Ansprüche 1 bis 3 definiert, worin $R_{16}$ ein Methylrest in Position alpha ist.

5. Verbindungen der Formel (I) wie in irgendeinem der Ansprüche 1 bis 4 definiert, worin $R_{17}$ ein Wasserstoffatom ist.

6. Verbindungen der Formel (I) wie in irgendeinem der Ansprüche 1 bis 5 definiert, worin die punktierte Linie in Position 1(2) die Anwesenheit einer zweiten Bindung anzeigt.

7. Verbindung der Formel (I) wie in irgendeinem der Ansprüche 1 bis 6 definiert, die der folgenden Formel entspricht:
   - 21-{4 [3,6-Bis-(Diethylamino)-2-pyridyl]-1-piperazinyl}-16alpha-methyl-pregna-1,4,9(11)-trien-3,20-dion und seine Salze.

8. Verfahren zur Herstellung der Verbindungen der Formel (I) wie in Anspruch 1 definiert, dadurch gekennzeichnet, daß man ein Produkt der Formel (II)

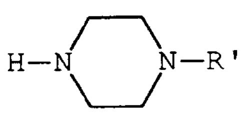

(II)

in der R' eine Schutzgruppe für die Aminofunktion darstellt, mit einem Produkt der Formel (III)

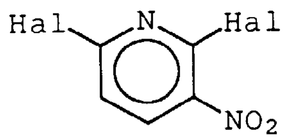

(III)

zur Reaktion bringt, in der Hal ein Halogenatom ist, um ein Produkt der Formel (IV)

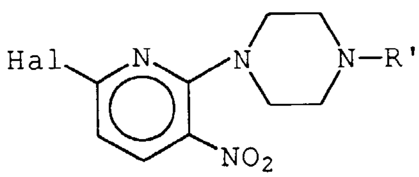

(IV)

zu erhalten, mit dem man ein Produkt der Formel (V)

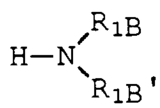

(V)

zur Reaktion bringt, in der $R_{1B}$ und $R_{1B}'$ entweder die oben für $R_B$ und $R_B'$ angegebene Bedeutung besitzen oder so sind, oder auch eines von ihnen eine monovalente Schutzgruppe für die Aminofunktion darstellt und das andere ein Wasserstoffatom ist, oder auch $R_{1B}$ und $R_{1B}'$ zusammen eine divalente Schutzgruppe darstellen, um ein Produkt der (VI)

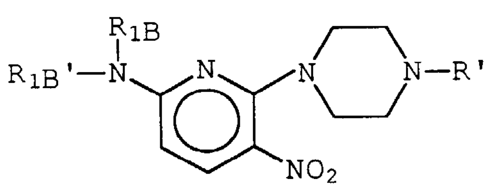

(VI)

zu erhalten, das man einer Hydrierungs-Reaktion unterzieht, um ein Produkt der Formel (VII)

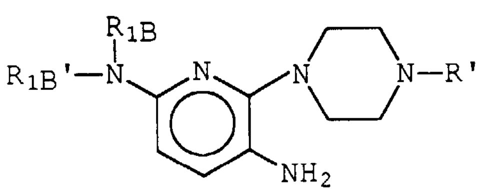

(VII)

zu erhalten, das man, wenn erwünscht
entweder a) der Einwirkung von einem oder zwei Äquivalenten eines Monohalogen-Derivates von $R_A$ oder $R_A'$ unterzieht, um ein Produkt der Formel (VII')

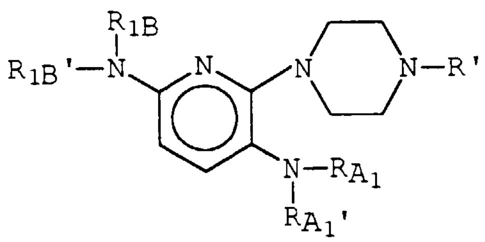

(VII')

zu erhalten, in der entweder eines von $R_{A1}$ und $R_{A1}'$ ein Wasserstoffatom und das andere einen Alkylrest darstellen oder beide den gleichen Alkylrest bedeuten,
<u>oder</u> b) der Einwirkung eines Monohalogen-Derivates von $R_A$ oder $R_A'$ und anschließend der Einwirkung eines Monohalogen-Derivates von $R_A'$ oder $R_A$ unterzieht, um ein Produkt der Formel (VII")

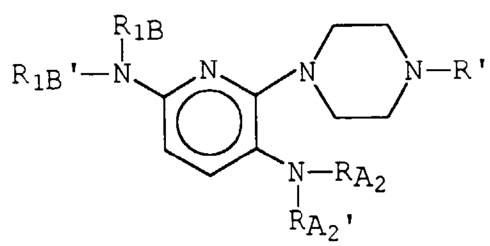

(VII'')

zu erhalten, in der $R_{A2}$ und $R_{A2}'$ unterschiedliche Alkylreste darstellen,
<u>oder</u> c) der Einwirkung eines Dihalogen-Derivates von Butan oder Pentan, gegebenenfalls substituiert durch einen Alkylrest mit 1 bis 3 Kohlenstoffatomen, unterzieht, um ein Produkt der Formel (VII''')

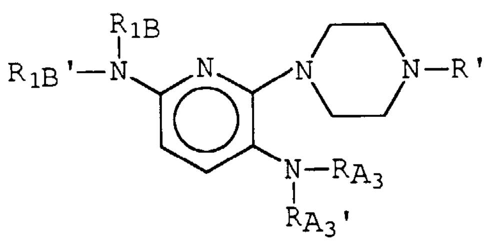

(VII''')

zu erhalten, in der $R_{A3}$ und $R_{A3}'$ zusammen mit dem Atom, an das sie gebunden sind, einen Heterocyclus mit 5 oder 6 Ringgliedern bilden,
und man die Produkte der Formeln (VII), (VII'), (VII") und (VII''') einer Reaktion zur Abspaltung der Schutzgruppe R' und, wenn erwünscht, der Gruppen $R_{1B}$ und/oder $R_{1B}'$ unterzieht, um eine Verbindung der Formel ($I_A$)

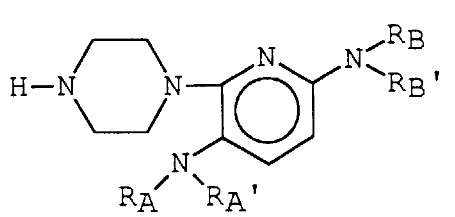

($I_A$)

zu erhalten, in der $R_A$, $R_A'$, $R_B$ und $R_B'$ die in Anspruch 1 angegebene Bedeutung besitzen, und man anschließend das Produkt der Formel ($I_A$), wenn erwünscht, in ein Salz umwandelt oder, wenn erwünscht, in einem neutralen Lösungsmittel und in Anwesenheit einer Base der Einwirkung einer Verbindung der Formel (VIII)

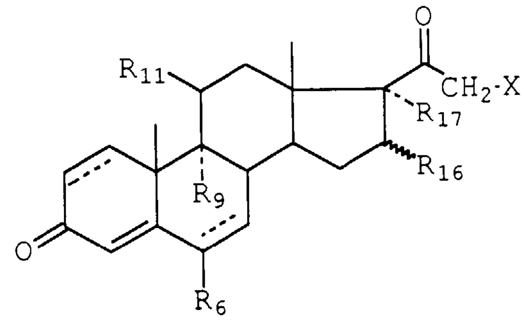

(VIII)

unterzieht, in der X ein Halogenatom ist und $R_6$, $R_9$, $R_{11}$, $R_{16}$, $R_{17}$, die punktierten Linien und die Wellenlinie die gleiche Bedeutung wie oben besitzen, um eine Verbindung der Formel (I)

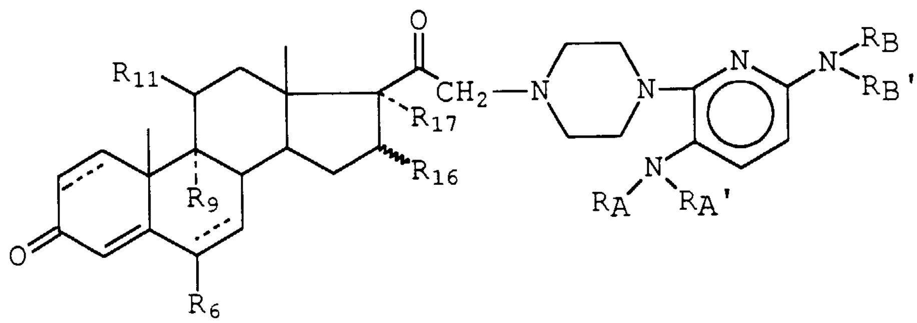

(I)

zu erhalten, und man, wenn erwünscht, die erhaltene Verbindung der Einwirkung einer Säure unterzieht, um das entsprechende Salz zu erhalten.

**9.** Als Arzneimittel die Verbindungen der Formel (I) wie in irgendeinem der Ansprüche 1 bis 6 definiert und ihre Salze mit pharmazeutisch akzeptablen Säuren.

**10.** Als Arzneimittel gemäß Anspruch 9 die in Anspruch 7 definierte Verbindung und ihre Salze mit pharmazeutisch akzeptablen Säuren.

**11.** Pharmazeutische Zusammensetzungen, die als Wirkstoff mindestens ein wie in irgendeinem der Ansprüche 9 bis 10 definiertes Arzneimittel umfassen.

**12.** Als neue industrielle Produkte die Verbindungen der Formeln (VII), (VII'), (VII"), (VII"') und ($I_A$).

**Claims**

**1.** The compounds of formula (I):

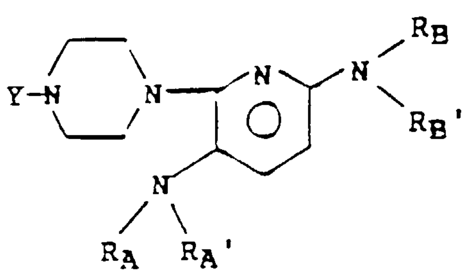

**(I)**

in which:

- $R_A$ and $R_A'$, $R_B$ and $R_B'$, identical or different, represent a hydrogen atom or an alkyl radical having 1 to 4 carbon atoms, it being understood that when $R_A$ and $R_A'$ are a hydrogen atom or a methyl radical, $R_B$ and $R_B'$ cannot be an ethyl radical; or form with the nitrogen atom to which they are linked a heterocycle with 5 or 6 members, optionally substituted by an alkyl radical having 1 to 3 carbon atoms,

and in which represents Y represents the radical

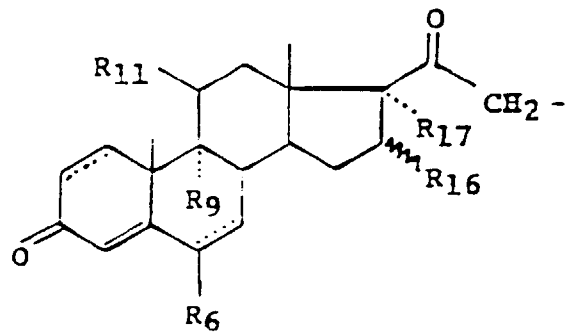

in which:

- $R_6$ represents a hydrogen atom, a methyl radical, a fluorine atom or a chlorine atom,
- $R_9$ and $R_{11}$ together form a second bond between carbons $C_9$ and $C_{11}$ or $R_9$ is a hydrogen atom or a fluorine atom and $R_{11}$ is a hydrogen atom, a hydroxyl radical or an oxo radical,
- $R_{16}$ represents a hydrogen atom or a methyl radical,
- $R_{17}$ represents a hydrogen atom, a hydroxyl radical or an acyloxy radical,
- the dotted lines, in rings A and B, indicate the optional presence of a second bond in position 1(2) and 6(7),
- the wavy line in position 16 indicates that the $R_{16}$ substituent can be found in alpha or beta position, and the salts of these compounds.

**2.** The compounds of formula (I) as defined in claim 1, in which $R_6$ is a hydrogen atom.

**3.** The compounds of formula (I) as defined in claim 1 or 2, in which $R_9$ and $R_{11}$ together form a second bond between the carbons which carry them.

**4.** The compounds of formula (I) as defined in any one of claims 1 to 3, in which $R_{16}$ is a methyl radical in alpha position.

**5.** The compounds of formula (I) as defined in any one of claims 1 to 4, in which $R_{17}$ is a hydrogen atom.

**6.** The compounds of formula (I) as defined in any one of claims 1 to 5, in which the dotted line in position 1(2) indicates the presence of a second bond.

**7.** The compound of formula (I) as defined in any one of claims 1 to 6, corresponding to the following formula:

- 21-[4-[3,6-bis(diethylamino) 2-pyridyl] 1-piperazinyl] 16alpha-methyl pregna-1,4,9(11)-triene-3,20-dione and its salts.

**8.** Preparation process for the compounds of formula (I), as defined in claim 1, characterized in that a product of formula (II):

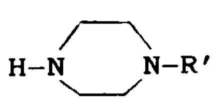

(II)

in which R' represents a protective group of the amino function, is reacted on a product of formula (III):

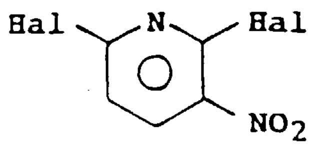

(III)

in which Hal represents a halogen atom, in order to obtain a product of formula (IV):

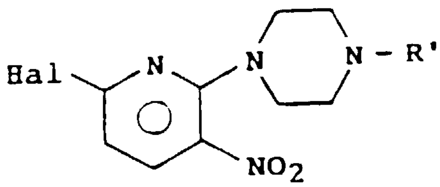

(IV)

on which a product of formula (V):

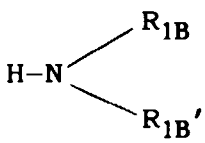

(V)

is reacted in which $R_{1B}$ and $R_{1B}$' either have the meaning indicated above for $R_B$ and $R_B$' or are such that, either one represents a monovalent protective group of the amino function, and the other represents a hydrogen atom, or $R_{1B}$ and $R_{1B}$' together represent a divalent protective group, in order to obtain a product of formula (VI):

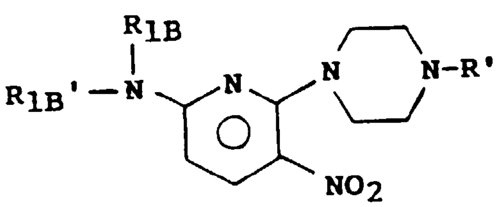

(VI)

which is subjected to a hydrogenation reaction in order to obtain a product of formula (VII):

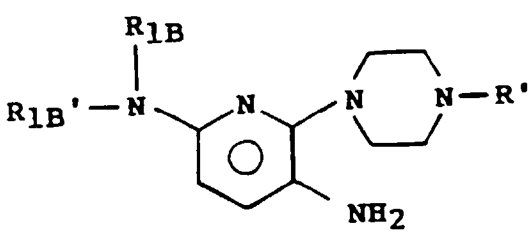

(VII)

which product, if desired
**either** a) is subjected to the action of one or two equivalents of a monohalogenated derivative of $R_A$ or of $R_A$' in order to obtain a product of formula (VII'):

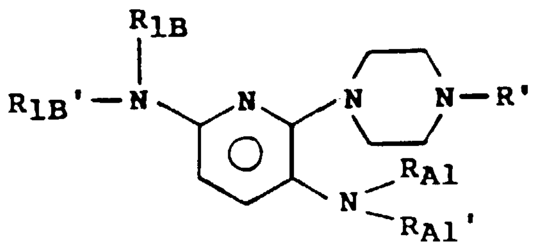

(VII')

in which either one of $R_{A1}$ and $R_{A1}'$ represents a hydrogen and the other represents an alkyl radical, or both represent the same alkyl radical,

**or** b) is subjected to the action of a monohalogenated derivative of $R_A$ or of $R_A'$, then to the action of a monohalogenated derivative of $R_A'$ or $R_A$ in order to obtain a product of formula (VII"):

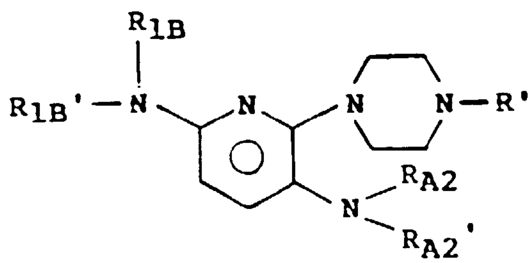

(VII'')

in which $R_{A2}$ and $R_{A2}'$ represent different alkyl radicals,

**or** c) is subjected to the action of a dihalogenated derivative of butane or pentane, optionally substituted by an alkyl radical having 1 to 3 carbon atoms, in order to obtain a product of formula (VII'''):

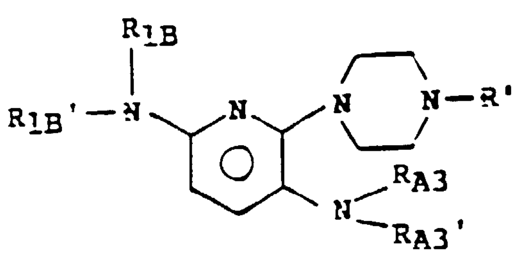

(VII''')

in which $R_{A3}$ and $R_{A3}'$ form with the atom to which they are linked a heterocycle with 5 or 6 members, which products of formulae (VII), (VII'), (VII") and (VII''') are subjected to a deblocking reaction of group R' and if desired groups $R_{1B}$ and/or $R_{1B}'$ in order to obtain a compound of formula ($I_A$):

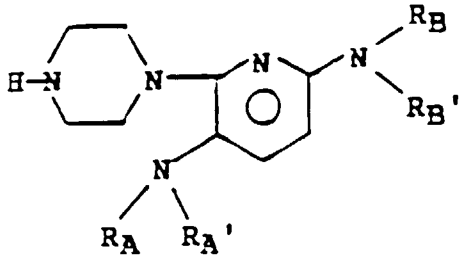

($I_A$)

in which $R_A$, $R_A'$, $R_B$ and $R_B'$ have the meaning indicated in claim 1, which product of formula ($I_A$) if desired is salified or if desired is subjected, in a neutral solvent and in the presence of a base, to the action of a compound of formula (VIII):

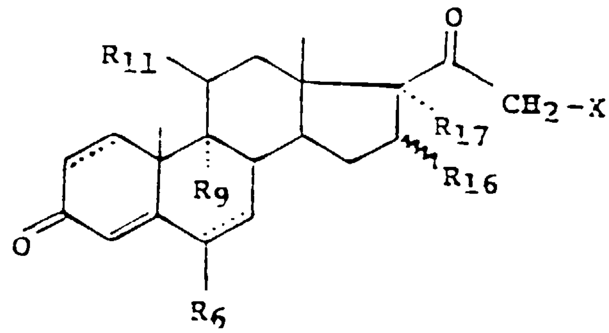

(VIII)

in which X is a halogen atom and $R_6$, $R_9$, $R_{11}$, $R_{16}$, $R_{17}$, the dotted lines and the wavy line have the same meaning as previously, in order to obtain a compound of formula (I):

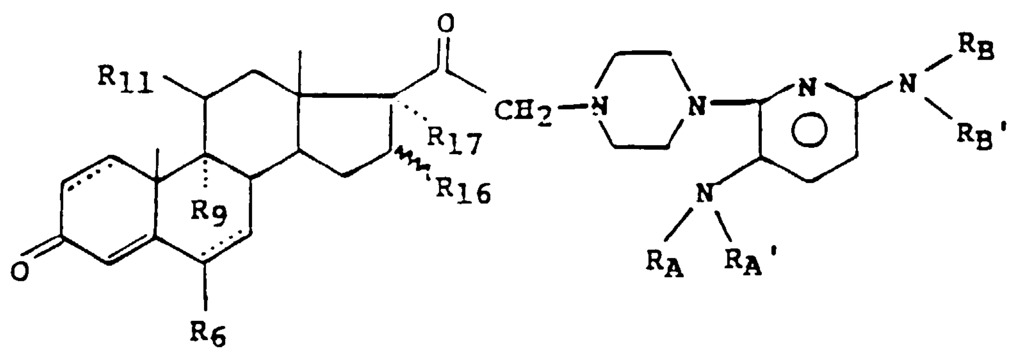

(I)

and if desired, the compound obtained is subjected to the action of an acid in order to obtain the corresponding salt.

**9.** As a medicament, the compounds of formula (I) as defined in any one of claims 1 to 6 and their salts with pharmaceutically acceptable acids.

**10.** As a medicament, according to claim 9, the compound defined in claim 7 and its salts with pharmaceutically acceptable acids.

**11.** The pharmaceutical compositions containing as active ingredient at least one medicament as defined in any one of claims 9 to 10.

**12.** As new industrial products, the compounds of formulae (VII), (VII'), (VII"), (VII"') and ($I_A$).